# EUROPEAN PATENT SPECIFICATION

(11) **EP 4 176 837 B1**
(45) Date of publication and mention of the grant of the patent: **27.11.2024**
(21) Application number: 22204678.1
(22) Date of filing: 31.10.2022
(51) Int. Cl.: A61C 19/04

(54) **BONE TREATMENT DEVICE**
KNOCHENBEHANDLUNGSVORRICHTUNG
DISPOSITIF DE TRAITEMENT OSSEUX

(30) Priority: 05.11.2021 JP 2021180977
(43) Date of publication of application: 10.05.2023
(73) Proprietor: J. Morita MFG. Corp., Kyoto-shi, Kyoto 612-8533 (JP); National University Corporation Hokkaido University, Sapporo-shi, Hokkaido 060-0808 (JP)
(72) Inventor: SUGAYA, Tsutomu, Sapporo-shi, Hokkaido, 060-0808 (JP); YAMADA, Keita, Sapporo-shi, Hokkaido, 060-0808 (JP); BAN, Shingo, Sapporo-shi, Hokkaido, 060-0808 (JP); DOKAI, Kei, Sapporo-shi, Hokkaido, 060-0808 (JP); UEDA, Tomoaki, Kyoto, 612-8533 (JP); YAMAGUCHI, Shunsuke, Kyoto, 612-8533 (JP); MATOBA, Kazunari, Kyoto, 612-8533 (JP)
(74) Representative: Müller Hoffmann & Partner

(56) References cited:
- US-A1- 2012 270 177
- US-A1- 2015 272 470
- US-A1- 2017 273 755
- IWASAKI A ET AL: "Differentiation of the observed low frequency (1200MHz) EPR signals in whole human teeth", APPLIED RADIATION AND ISOTOPES, ELSEVIER, OXFORD, GB, vol. 62, no. 2, 1 February 2005 (2005-02-01), pages 133 - 139, XP027716097, ISSN: 0969-8043, [retrieved on 20050201]

## Description

### BACKGROUND OF THE INVENTION

### Field of the Invention

The present disclosure relates to a treatment device that passes a high frequency current through a site to be treated, and particularly to a treatment device that passes a high frequency current for promoting bone formation.

### Description of the Background Art

In the field of dentistry, a dental root canal treatment is sometimes conducted so as to remove the dental pulp, or control inflammation of the root apex. In a conventional root canal treatment, the treatment has been performed by cutting and enlarging a root canal with a reamer and a file and removing contaminating tissues and contaminants in the root canal, and then filling the root canal with a pharmaceutical.

However, the shape of a root canal is complicated, and the shape differs depending on the kind of the tooth and the individual. Therefore, in a root canal treatment, there is a part where enlargement of root canal with a reamer and a file is difficult, and an inflammatory factor remaining in the part where enlargement of the root canal is impossible would cause inflammation after the operation. Also, a bone defect can occur in the apical area, or a root apex lesion can occur, and treatments for these are also involved in a root canal treatment. Therefore, in a root canal treatment, it has been common that regeneration of the defected or lesioned bone is an index for determination of healing.

International Publication No. 2008/114244 discloses a treatment device that conducts a root canal treatment for reducing inflammatory factors, bacteria and the like in a root canal of tooth. In the treatment device, an electrode to be inserted into a root canal is included in a dental tool that communicates with a measuring device that measures the position of the root apex. Further, the dental tool is capable of communicating with a unit that applies an electric pulse. Therefore, the treatment device is capable of applying an electric pulse to the root canal via the electrode inserted into the root canal, and reducing inflammatory factors, bacteria and the like in a root canal by the applied electric pulse.

Furthermore, studies for promoting bone regeneration by utilizing a faint current have been made, and a DC current stimulation method (DC method), an AC current stimulation method (AC method), a capacity coupling type electrostimulation method (CCEF method) and the like are known (Yumoto H, Hirao K, Tominaga T, Bando N, Takahashi K, Matsuo T "Electromagnetic wave irradiation promotes osteoblastic cell proliferation and up-regulates growth factors via activation of the ERK1/2 and p38 MAPK pathways" Cell Physiol Biochem, 35: 601-615, 2015).

### SUMMARY OF THE INVENTION

However, in the root canal treatment, when a bone defect or a root apex lesion is generated in the apical area, healing is not determined until the defected or lesioned bone is regenerated, so that the time period until healing is determined is extended if a relatively long time is required for regeneration of the bone. Of course, in the root canal treatment, it is desired to shorten the time until achievement of healing because the patient feels an unpleasantness until healing is achieved.

The present disclosure was devised to solve the aforementioned problem, and it is an object of the present invention defined in the appended claims to provide a treatment device capable of shortening the time period until healing is determined even when a bone defect or a root apex lesion is generated in the apical area.

The treatment device according to the present invention is a treatment device that passes a high frequency current through a site to be treated. The treatment device includes a holder that holds an electrode to be located in a site to be treated, a power supply that passes a high frequency current through the electrode, and a controller that controls a frequency of the high frequency current to be passed through the electrode from the power supply. The controller controls the frequency of the high frequency current to be passed through the electrode to fall within a range of 1.001 MHz to 11.700 MHz.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

Fig. 1 is an external appearance view of a treatment device.
Fig. 2 is a block diagram showing a configuration of the treatment device of Fig. 1.
Fig. 3A is a schematic view for illustrating a method of regenerating bone in an apical area.
Fig. 3B is a schematic view for illustrating the method of regenerating bone in the apical area.
Fig. 4 is a chart showing a waveform of a high frequency current in a bone regeneration mode to be passed through the electrode of the treatment device of Fig. 1.
Fig. 5 is a view for illustrating an experiment in which bone regeneration is conducted in the treatment device o Fig. 1.
Fig. 6 is a view showing an experimental result of the experiment of regenerating bone conducted in Fig. 5.
Fig. 7 is a chart showing a waveform of a high frequency current to be passed through an electrode of another treatment device.
Fig. 8 is a flowchart for illustrating control of the treatment device of Fig. 7.

### DESCRIPTION

Hereinafter, the present disclosure will be described by referring to the drawings.

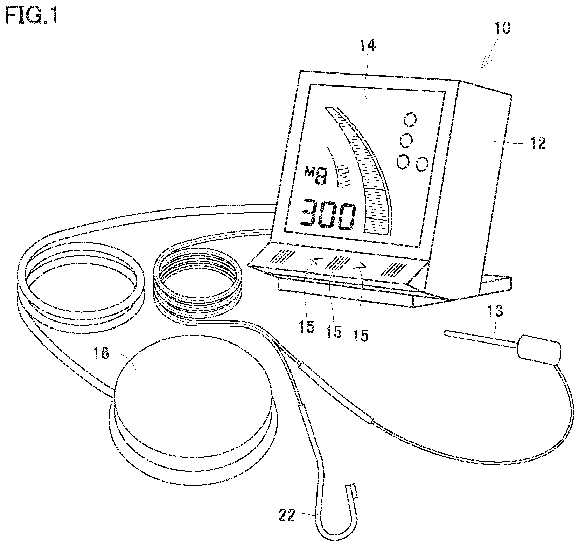

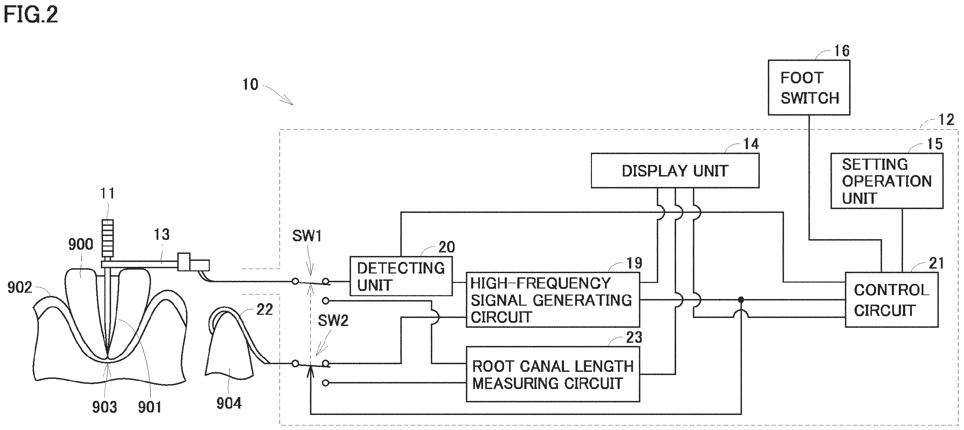

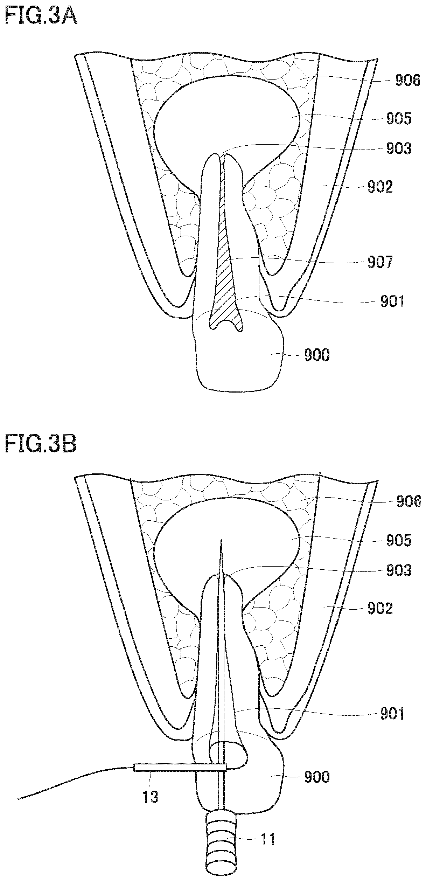

Fig. 1 is an external appearance view of a treatment device Fig. 2 is a block diagram showing a configuration of the treatment of Fig. 1. Figs. 3A and 3B each are a schematic view for illustrating a method of regenerating bone in an apical area. In the apical area, it is sometimes the case that a bone defect or a root apex lesion is generated, and in a tooth 900 shown in Fig. 3A, a bone defect 905 is generated in alveolar bone 906 near a root apex 903. In a root canal treatment, a significant time is required and the time period until healing is determined is elongated if one waits for natural regeneration of the bone of the part of bone defect 905.

Here, in the treatment device 10 of Fig. 1, as in tooth 900 shown in Fig. 3B, a root canal 901 is cut and enlarged with a cutting tool such as a reamer and a file, and a dental pulp 907 or an inflammatory factor in root canal 901 is removed, and then an electrode (file 11) is inserted in root canal 901, and a high frequency current is passed through the part of bone defect 905 which is a site to be treated.

The treatment device 10 is capable of promoting bone regeneration of the part by passing a high frequency current of a specific frequency through the part where bone defect 905 is generated (bone regeneration mode). In particular, in the present disclosure, it was newly found that bone regeneration in the part where bone defect 905 is generated is further promoted by controlling the frequency of the high frequency current to be passed through the electrode to fall within a range of 1.001 MHz to 11.700 MHz. More preferably, it was newly found that bone regeneration is further promoted by controlling the frequency of the high frequency current to be passed through the electrode to fall within a range of 7.751 ± 2 MHz. By passage of a high frequency current having such a frequency, it is possible to promote bone regeneration not only in the part where bone defect 905 is generated, but also in the part where a root apex lesion is generated similarly. The high frequency current to be passed through the electrode in the bone regeneration mode is preferably a high frequency current of a sinusoidal wave. Of course, the high frequency current to be passed through the electrode in the bone regeneration mode is not necessarily limited to a high frequency current of a sinusoidal wave.

In treatment device 10, besides passage of a high frequency current for promoting bone regeneration (bone regeneration mode), it is also possible to pass a high frequency current to cauterize and sterilize inflammatory factors, bacteria and the like remaining in root canal 901 by Joule heat after cutting and enlarging root canal 901 with a cutting tool (sterilization mode). Treatment device 10 need not achieve perfect sterilization, but only need to heat denature the dental pulp and granulation to necrose or deactivate them by passage of a high frequency current. The treatment of sterilizing inside root canal 901 by passage of a high frequency current is also called EMAT (Electro-Magnetic Apical Treatment), and is disclosed, for example, in a literature (for example, Naoki BANDO, Toshihiko TOMINAGA, Takashi SUMITOMO, Saki HIRAO, Koji HIRAO, Takashi MATSUO, "Application of electromagnetic radiation to endodontics - EMAT (Electro-Magnetic Apical Treatment)", 2011, The Journal of Japan Endodontic Association, vol. 32, p. 184-200). This disclosure reports that passage of a high frequency current through the affected part before and after treatment reduces inflammatory factors, bacteria and the like in the site to be treated and results in very excellent prognosis.

Specifically, as shown in Fig. 1, treatment device 10 includes a unit 12 that passes a high frequency current through a file 11 which is a cutting tool (see Fig. 2), and a file holder 13 for holding file 11, and is capable of passing a high frequency current through file 11 attached to the tip end of file holder 13. In this disclosure, file 11 is attached to the tip end of file holder 13, and this file 11 serves as an electrode to be located in a site to be treated, however, file holder 13 and file 11 may be integrated.

File holder 13 is formed by an approximately bar-like casing, and is capable of holding a metallic part of file 11. File holder 13 is capable of electrically connecting the file and unit 12 by holding the metallic part of file 11. Unit 12 is provided with a display unit 14 and a setting operation unit 15, and is connected with a foot switch 16 and a passive electrode 22.

As shown in Fig. 2, unit 12 is provided with a high-frequency signal generating circuit 19, a detecting unit 20, a control circuit 21, a root canal length measuring circuit 23, and a switch SW1 to a switch SW2 besides display unit 14 and setting operation unit 15.

Setting operation unit 15 is a setting button provided to set the operation of treatment device 10. In setting operation unit 15, a current value, a frequency, an energization period of a high frequency current to be passed through file 11, a display setting of display unit 14 and the like can be set. Here, the energization period is a time of one passage when energization with high frequency current by one operation is conducted by one-shot, and is a sum of times of plural passages when energization with high frequency current by one operation is divisionally conducted by the plural passages.

Foot switch 16 is an operational unit provided for operating passage of high frequency current, and a control signal is transmitted to high-frequency signal generating circuit 19 from control circuit 21 by being pressed down by a user. High-frequency signal generating circuit 19 passes a high frequency current set in setting operation unit 15 through file 11 according to the received control signal.

High-frequency signal generating circuit 19 passes a high frequency current having, for example, a frequency of 300 kHz to 1000 kHz (second range) and a current value of 20 mA to 200 mA (a range required in a root canal treatment for reducing inflammatory factors, bacteria and the like in a root canal) between file 11 and passive electrode 22 in the sterilization mode. Also, high-frequency signal generating circuit 19 passes a high frequency current having, for example, a frequency of 1.001 MHz to 11.700 MHz (first range) and a current value of 10 µA to less than 20 mA (a range required in a root canal treatment for promoting bone regeneration in the site where a bone defect is generated in the root canal or in the root apex) between file 11 and passive electrode 22 in the bone regeneration mode. In high-frequency signal generating circuit 19, the frequency of the high frequency current to be passed through file 11 in sterilization mode (second range) is different from the frequency of the high frequency current to be passed through file 11 in the bone regeneration mode (first range). Of course, the frequency and the current value of the high frequency current that can be generated in high-frequency signal generating circuit 19 are not limited to the frequency and the current value described above.

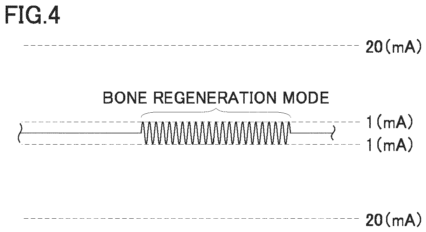

Fig. 4 is a chart showing a waveform of a high frequency current in the bone regeneration mode to be passed through the electrode of the treatment device of Fig. 1. The chart shown in Fig. 4 is a conceptual diagram, and the illustrated waveform and wave number are different from the waveform and the wave number of an actual high frequency current. This also applies to the waveform of the high frequency current as follows. The user switches to the bone regeneration mode from the sterilization mode in setting operation unit 15, and operates foot switch 16 in the part of bone defect 905 which is a site to be treated to cause high-frequency signal generating circuit 19 to pass a high frequency current through file 11. The waveform shown in Fig. 4 is a waveform when high-frequency signal generating circuit 19 is driven in the bone regeneration mode, and is a waveform when a high frequency current of a sinusoidal wave having a frequency (for example, 7.751 MHz) and a current value (for example, 1 mA) required for promoting bone regeneration is passed through a part of bone defect 905.

The current value, the frequency, the energization period and the like of the high frequency current to be output from high-frequency signal generating circuit 19 can be set by operating setting operation unit 15. Control circuit 21 controls high-frequency signal generating circuit 19 on the basis of the frequency and the current value detected by detecting unit 20 so that high-frequency signal generating circuit 19 can output the high frequency current having the frequency and the current value set in setting operation unit 15. However, when control circuit 21 does not control high-frequency signal generating circuit 19 on the basis of the frequency and the current value by detecting unit 20, treatment device 10 need not be provided with detecting unit 20. For passage of a high frequency current, file 11 is inserted into root canal 901, the tip end of file 11 is brought into contact with, for example, a part of bone defect 905, and passive electrode 22 is brought into contact with a part of the patient body such as a gingiva 902 or a lip 904. File holder 13 is a holding unit that holds file 11 which is an electrode to be located in a site to be treated, and high-frequency signal generating circuit 19 is a power supply unit that passes a high frequency current through the electrode.

Detecting unit 20 is a detector that detects a frequency and a current value of a high frequency current actually flowing in file 11 when a high frequency current is passed through file 11 from high-frequency signal generating circuit 19. Control circuit 21 conducts control of a frequency and a current value of a high frequency current to be passed through file 11 for high-frequency signal generating circuit 19 on the basis of a frequency and a current value detected in detecting unit 20. Control circuit 21 is provided with, as a hardware configuration, for example, a CPU (Central Processing Unit), a storage for storing programs, data and the like for executing the processing by the CPU, a RAM (Random Access Memory) functioning as work area of the CPU, a GPU (Graphics Processing Unit) mainly performing image processing, and an I/O interface for keeping the conformance of signals with peripheral equipment. The storage includes a storage unit such as nonvolatile memory provided inside control circuit 21, and a storage unit connected via a network.

Root canal length measuring circuit 23 flows a signal for measuring root canal length between file 11 and passive electrode 22 to measure a position of the tip end of file 11. Specifically, root canal length measuring circuit 23 applies two voltages having different frequencies between file 11 and passive electrode 22 to determine respective values of impedance, and identifies the position of the tip end of file 11 from root apex 903 according to a difference, a ratio or the like between these two values (actually values of voltage or current corresponding to the values of impedance). The measuring method of root canal length measurement is not limited to that described above, but various techniques including conventionally proposed measuring methods can be utilized. Also, in root canal length measurement, passive electrode 22 is brought into contact with a part of the patient body such as gingiva 902 or lip 904.

Switch SW1 is provided to switch the electric connection between file 11, and high-frequency signal generating circuit 19 or root canal length measuring circuit 23. Also, switch SW2 is provided to switch the electric connection between passive electrode 22, and high-frequency signal generating circuit 19 or root canal length measuring circuit 23.

Switching between switch SW1 and switch SW2 is realized by control circuit 21 on the basis of input information from setting operation unit 15. Specifically, control circuit 21 controls switch SW1 and switch SW2 such that file 11 and passive electrode 22 are connected with high-frequency signal generating circuit 19 when a high frequency current is passed through file 11. Also, control circuit 21 controls switch SW1 and switch SW2 such that file 11 and passive electrode 22 are connected with root canal length measuring circuit 23 when a signal for root canal length measurement is flown between file 11 and passive electrode 22. Here, switch SW1 and switch SW2 are controlled in accordance with the operation mode set in setting operation unit 15 to switch the connecting destinations of file 11 and passive electrode 22. Switching between switch SW1 and switch SW2 may be linked with ON/OFF operation of foot switch 16.

Here, high-frequency signal generating circuit 19 and root canal length measuring circuit 23 are electrically separated by switch SW1 and switch SW2. Therefore, while a high frequency current is output to file 11, root canal length measuring circuit 23 is electrically disconnected from file 11 and passive electrode 22 by switch SW1 and switch SW2. Therefore, a high frequency current is not passed through root canal length measuring circuit 23, and it is possible to prevent root canal length measuring circuit 23 from breaking by a high frequency current.

Also, it is possible to know where the tip end position of file 11 is located from the root apex by root canal length measuring circuit 23. Therefore, the user is capable of switching from the root canal length measuring mode to the high-frequency energization mode by operating setting operation unit 15 after confirming that the tip end position of file 11 in the root canal has reached a position where the high frequency current is to be passed (for example, the part where bone defect 905 or a root apex lesion is generated) by root canal length measuring circuit 23. Therefore, in treatment device 10, it is possible to pass a high frequency current through file 11 while the tip end position of file 11 is kept at an appropriate position. Of course, treatment device 10 may be configured only to pass a high frequency current through file 11 without provision of root canal length measuring circuit 23, and a high frequency current may be passed through file 11 while the tip end position of file 11 is confirmed with other root canal length measuring device.

Display unit 14 is configured, for example, by a liquid crystal display, and displays a current value being passed through file 11 by high-frequency signal generating circuit 19, a position of the tip end of file 11 measured by root canal length measuring circuit 23, and so on, and functions as an alarm unit that notifies necessary information of a user of treatment device 10. Display unit 14 may notify, for example, of information that the frequency and the current value of the high frequency current being passed are out of the ranges of the frequency and the current value required for the root canal treatment when the frequency and the current value detected by the detecting unit 20 become out of the set ranges of frequency and current value. Display unit 14 may be configured by an organic EL display, electronic paper, a light-emitting diode or the like. The alarm unit may be a lamp or a speaker (not shown) besides display unit 14, and may notify the user by lightening of the lamp, or may notify the user by outputting buzzer sound from the speaker.

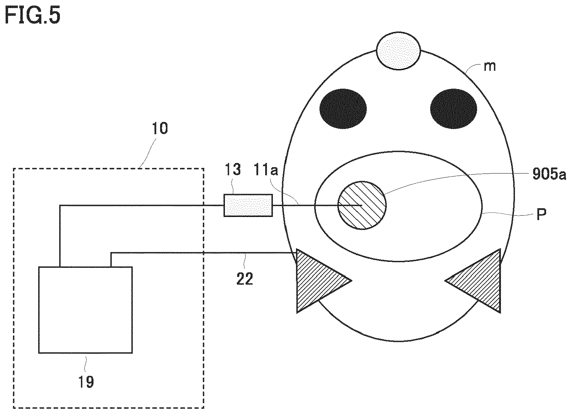

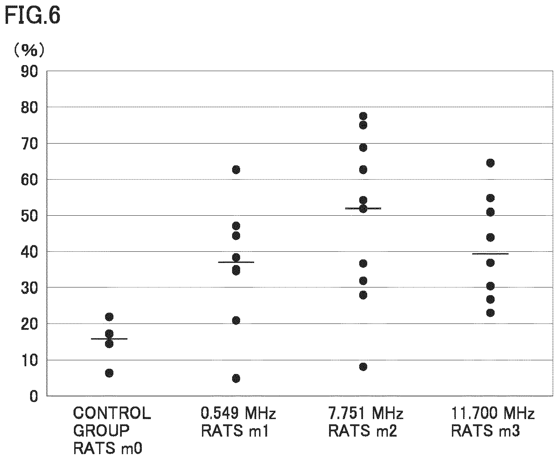

Treatment device 10 controls the frequency of the high frequency current to be passed through the electrode to fall within a range of 1.001 MHz to 11.700 MHz for promoting bone regeneration in the part where bone defect 905 or a root apex lesion is generated. More preferably, treatment device 10 controls the frequency of the high frequency current to be passed through the electrode to fall within a range of 7.751 ± 2 MHz. Promotion of bone regeneration caused by passing the high frequency current having such a frequency through the site to be treated is specifically described. Fig. 5 is a view for illustrating an experiment in which bone regeneration is conducted with the treatment device 10. Fig. 6 is a view showing an experimental result of the experiment of regenerating bone conducted in Fig. 5.

In the experiment shown in Fig. 5, the experiment was conducted using a rat skull instead of a human tooth. In this experiment, the scalp of a rat m shown in Fig. 5 is incised to expose part of the skull P, and a bone defect 905a is prepared in the exposed skull P at one site on the left of rat m with a trephine bar of 4.8 mm in outside diameter. A high frequency current is passed through prepared bone defect 905a with treatment device 10. Specifically, treatment device 10 passes a high frequency current through an electrode 11a from high-frequency signal generating circuit 19 in the condition that electrode 11a held by file holder 13 is located in an appropriately center part of bone defect 905a, and passive electrode 22 is stuck in the skin near the left ear of rat m. In this experiment, a needle instead of file 11 was used as electrode 11a.

A plurality of prepared rats m were subjected to the experiment while being divided into the following groups: rats m1 through which a high frequency current of 0.549 MHz is passed; rats m2 through which a high frequency current of 7.751 MHz is passed, rats m3 through which a high frequency current of 11.700 MHz is passed, and rats m0 (control group) through which a high frequency current is not passed. For each rat m, passage of a high frequency current is conducted predetermined times at predetermined intervals. From the day on which electrode 11a was attached, the incised scalp was returned, and electrode 11a was stuck in the scalp of the approximately center part of bone defect 905a and a high frequency current was passed. The current value of the high frequency current to be passed was 1 mA.

The graph of Fig. 6 shows the result of the experiment shown in Fig. 5. The vertical axis of the graph indicates hard tissue formation rate. Letting an area of prepared bone defect 905a be A, and a defective area after passage of a high frequency current predetermined times be DA, a hard tissue formation rate can be defined as (A-DA)/A. The hard tissue formation rate is an equivalent value of a bone regeneration rate. For measurement of defective area DA, a µCT device was used.

As in the graph of Fig. 6, rats m1 (sample number n = 7) through which a high frequency current of 0.549 MHz was passed showed a hard tissue formation rate within a range of about 5% to about 62% with a median value of about 38%. Rats m2 (sample number n = 10) through which a high frequency current of 7.751 MHz was passed showed a hard tissue formation rate within a range of about 9% to about 79% with a median value of about 52%. Rats m3 (sample number n = 8) through which a high frequency current of 11.700 MHz was passed showed a hard tissue formation rate within a range of about 22% to about 65% with a median value of about 40%. Rats m0 (control group) (sample number n = 4 through which a high frequency current was not passed showed a hard tissue formation rate within a range of about 6% to about 22% with a median value of about 15%. A median value is indicated by a lateral bar in the graph of Fig. 6.

As is apparent from the experimental result shown in Fig. 6, by passing a high frequency current having a frequency ranging from 0.549 MHz to 11.700 MHz through the electrode, the hard tissue formation rate (= bone regeneration rate) is increased in comparison with the case where a high frequency current is not passed. It is found that the hard tissue formation rate is most increased especially by passing a high frequency current having a frequency of 7.751 MHz through the electrode. In other words, the frequency most contributing to bone regeneration among the frequencies of high frequency current to be passed through the electrode is 7.751 MHz.

Here, when the frequency varies from 7.751 MHz to 11.700 MHz, the hard tissue formation rate (median value) varies from about 52% to about 40%. Assuming that the relationship between the hard tissue formation rate and the frequency linearly changes, a variation of 1 MHz in the frequency will result in a decrease of about 3% in the hard tissue formation rate (median value). This indicates that the frequency can be controlled to the range of 7.751 ± 2 MHz so as to ensure the hard tissue formation rate (median value) of greater than or equal to about 45%.

In the treatment device 10, since a high frequency current having a frequency within a range of 300 kHz to 1000 kHz is used in the sterilization mode, it is preferred to control the frequency used in the bone regeneration mode to fall within a range of 1.001 MHz to 11.700 MHz.

As described above, the treatment device 10 includes file holder 13 that holds file 11 to be located in a site to be treated (for example, bone defect 905 or the like), high-frequency signal generating circuit 19 that passes a high frequency current through file 11, and control circuit 21 that controls a frequency of a high frequency current to be passed through file 11 from high-frequency signal generating circuit 19. Control circuit 21 controls the frequency of the high frequency current to be passed through file 11 to fall within a range of 1.001 MHz to 11.700 MHz. Preferably, the frequency of the high frequency current is controlled to fall within a range of 7.751 ± 2 MHz.

In the treatment device 10 configured as described above, since the control circuit 21 controls the frequency of the high frequency current to be passed through the electrode to fall within a range of 1.001 MHz to 11.700 MHz (preferably, a range of 7.751 ± 2 MHz), it is possible to promote bone regeneration and shorten the period until healing is determined even when a bone defect or a root apex lesion is generated in the apical area.

In the treatment device 10 described above a high frequency current passes through file 11 in the site to be treated while the user switches between the sterilization mode and the bone regeneration mode. It is, however, also possible that the treatment device operates first in the sterilization mode of sterilizing a root canal, and then automatically switches the mode and operates in the bone regeneration mode for promoting bone regeneration. Also in this case, the configuration of the treatment device 10 described above is employed, and the same constituent is denoted by the same reference numeral, and detailed description thereof is not repeated.

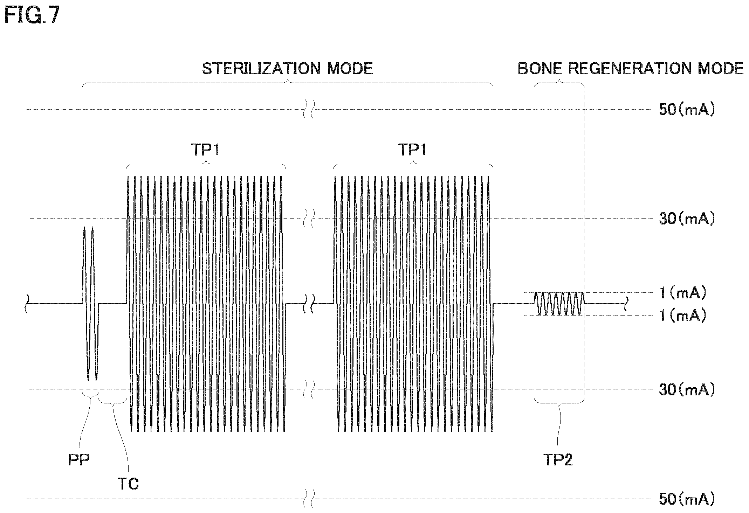

Fig. 7 is a chart showing a waveform of a high frequency current to be passed through an electrode of the treatment device 10 in this case. A user determines the position where a high frequency current is to be passed on the basis of root canal length measuring circuit 23, and passes the high frequency current through file 11 from high-frequency signal generating circuit 19 by operating foot switch 16 at this energization position. The treatment device 10 operates first in the sterilization mode of sterilizing a root canal as a high-frequency energization mode.

In the operation in the sterilization mode, before actual passage of a high frequency current through file 11 from high-frequency signal generating circuit 19, a high frequency current is preliminarily passed through file 11. The energization in a preliminary period (PP) shown in Fig. 7 is preliminary energization, and actual energization is conducted in the following first period (TP1). In the energization of the first period, a high frequency current having a current value (for example, 30 mA to 50 mA) in the range of current value required for a root canal treatment for reducing inflammatory factors, bacteria and the like in a root canal is passed. In the energization of the preliminary period, by passing a current value that is smaller than the minimum current value (for example, 30 mA) of current values within the range of current value required for the root canal treatment as a preliminary current, pain or the like in the energization of the preliminary period can be mitigated. In the preliminary period and in the first period, a high frequency current having a frequency within a range of 300 kHz to 1000 kHz (second range) is passed.

High-frequency signal generating circuit 19 includes a constant voltage circuit, and passes a high frequency current through file 11 by constant voltage control. Therefore, by applying, in the preliminary period, a high-frequency voltage that is lower than the high-frequency voltage to be applied in the first period, high-frequency signal generating circuit 19 can pass, in the preliminary period, a current value that is smaller than the current value to be passed in the first period. High-frequency signal generating circuit 19 may be configured by a constant current circuit, and may pass a high frequency current through file 11 by constant current control.

Control circuit 21 controls so that the current value of the high frequency current to be flown to file 11 from high-frequency signal generating circuit 19 in energization of the first period falls within a predetermined range, on the basis of the current value detected by detecting unit 20 when the preliminary current is flown to file 11. In accordance with the difference or ratio between the assumed current value of preliminary current and the current value detected by detecting unit 20, control circuit 21 changes the optimum voltage with which the current value of the high frequency current to be flown in file 11 falls within the predetermined range, and applies the voltage to high-frequency signal generating circuit 19. That is, control circuit 21 optimizes the voltage to be applied to high-frequency signal generating circuit 19 so that the current value of the high frequency current to be flown in file 11 falls within the predetermined range. The current value of the high frequency current to be flown in file 11 is not limited to the current value required for a root canal treatment for reducing inflammatory factors, bacteria and the like in a root canal. In the above description, control circuit 21 compares the current value of the preliminary current and the current value detected by detecting unit 20 by difference, however, the comparison may be conducted by other comparing means such as ratio or the like.

High-frequency signal generating circuit 19 conducts energization of, for example, 0.02 seconds (20 ms) as the preliminary period, followed by a rest period (TC) of, for example, 0.03 seconds (30 ms), and conducts energization of, for example, 0.07 seconds (70 ms) as the first period. High-frequency signal generating circuit 19 repeats the period of the first period and the rest period (total 0.1 second (100 ms) N times (for example, 10 times) for one energization (for example, one operation of foot switch 16). In treatment device 10, as the user presses down foot switch 16 once, a high frequency current is passed through file 11 from high-frequency signal generating circuit 19 for about 1 second (including the rest period). The number of repetitions of control of the rest period and the first period can be set in advance in setting operation unit 15 by the user, and is set, for example, to 10. Of course, the number of repetitions of control of the rest period and the first period may be set to a value other than 10. Also, in setting operation unit 15, an energization period (for example, 1 second) may be set rather than setting the number of repetitions of control of the rest period and the first period.

Next, treatment device 10 operates in the sterilization mode, and then operates in the bone regeneration mode. In the bone regeneration mode, a high frequency current having a frequency of 1.001 MHz to 11.700 MHz (first range) that is different from the frequency of the high frequency current passed in the sterilization mode (second range) is passed. Also, in the bone regeneration mode, a high frequency current having a smaller current value (for example, 10 µA to less than 20 mA) compared with the current value (for example, 30 mA to 50 mA) of the high frequency current passed in the sterilization mode is passed through file 11. Energization of the second period (TP2) shown in Fig. 7 is energization in the bone regeneration mode. In Fig. 7, while the length of the second period is described as being shorter than the length of the first period, the second period may be a length of about several seconds to about 5 seconds. Also, control circuit 21 may determine the voltage to be applied by high-frequency signal generating circuit 19 such that the current value of the high frequency current to be flown into file 11 from high-frequency signal generating circuit 19 in energization of the second period is a tiny current value on the basis of the current value detected by detecting unit 20 when the preliminary current is flown into file 11.

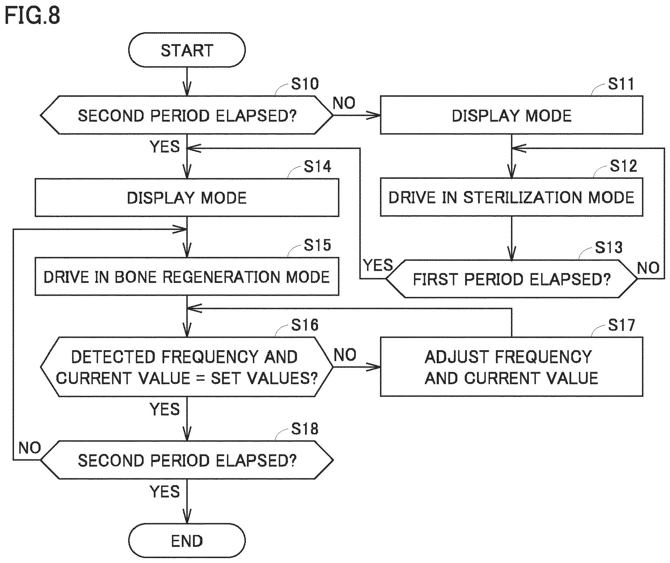

Next, control of the high frequency mode to be passed through file 11 in treatment device 10 is described by using a flowchart. Fig. 8 is a flowchart for illustrating control of the treatment device. First, a user determines the position (site to be treated) where a high frequency current is to be passed on the basis of root canal length measuring circuit 23, and operates treatment device 10 in the high frequency mode by operating foot switch 16 at this energization position. Control circuit 21 determines whether or not the energization period is the second period (step S10). When the energization period is not the second period (NO in step S10), control circuit 21 determines that the energization period is the first period and the operation mode is the sterilization mode, and notifies display unit 14 of information of executing the sterilization mode (step S11). Control circuit 21 displays the operation mode to be executed in step S11, and passes a high frequency current in the sterilization mode shown in Fig. 7 through file 11 (step S12).

Control circuit 21 determines whether or not the energization period is the first period (including the preliminary period and the rest period) (step S13). When the energization period is the first period (Yes in step S13), control circuit 21 returns the process to step S12, and passes a high frequency current in the sterilization mode through file 11. Meanwhile, when the energization period is not the first period (NO in step S13), control circuit 21 determines that the energization period is the second period and the operation mode is the bone regeneration mode, and notifies display unit 14 of information of executing the bone regeneration mode (step S14). When the energization period is the second period (Yes in step S10), control circuit 21 advances the process to step S14.

Control circuit 21 displays the operation mode to be executed in step S14, and passes a high frequency current in the bone regeneration mode shown in Fig. 7 through file 11 (step S15). Here, the frequency and the current value to be passed in the bone regeneration mode are set values determined in advance, and can be set by a user with setting operation unit 15 or the like.

In order to promote bone regeneration, it is required that the frequency and the current value of the high frequency current to be passed through file 11 in the second period conform to the set values. Here, the set values are, for example, a value of frequency ranging from 1.001 MHz to 11.700 MHz (first range), and a value of current value ranging from 10 µA to less than 20 mA. Here, control circuit 21 determines whether or not the frequency and the current value detected by detecting unit 20 conform to the set values (step S16). When the frequency and the current value detected by detecting unit 20 do not conform to the set values (NO in step S16), control circuit 21 controls high-frequency signal generating circuit 19 to adjust the frequency and the current value of the high frequency current to be passed through file 11 to conform to the set values (step S17). Control circuit 21 adjusts the high frequency current to be passed in step S17, and then returns the process to step S16 to perform feedback control. Control circuit 21, when feedback controlling the frequency and the current value of the high frequency current, may control display unit 14 to display that the frequency and the current become out of the above ranges when the frequency and the current value detected by detecting unit 20 vary to become out of the above ranges.

When the frequency and the current value detected by detecting unit 20 approximately conform to the set values (YES in step S16), control circuit 21 determines whether or not the second period has elapsed in the energization period (step S18). When the second period has not elapsed in the energization period (NO in step S18), control circuit 21 returns the process to step S15. On the other hand, when the second period has elapsed in the energization period (YES in S18), control circuit 21 ends the operation in the high frequency mode.

As described above, the treatment device 10 divides the one energization period in which control circuit 21 passes a high frequency current through file 11 into a plurality of periods, and the energization period includes the first period of passing a high frequency current having a frequency controlled to fall within the second range of 300 kHz to 1000 kHz through file 11, and the second period of passing a high frequency current having a frequency controlled to fall within the first range of 1.001 MHz to 11.700 MHz through file 11. As a result, treatment device 10 can automatically switch the mode and operate in the bone regeneration mode of promoting bone regeneration after operating in the sterilization mode.
(a) It has been described that the treatment device 10 passes a high frequency current having a frequency ranging from 1.001 MHz to 11.700 MHz (first range) and a current value ranging from 10 µA to less than 20 mA between file 11 and passive electrode 22. However, the treatment device 10 may be a treatment device that applies an electromagnetic wave to a site to be treated. In the treatment device, file 11 is not used as an electrode for passage of a high frequency current, but file 11 is used as an antenna for application of an electromagnetic wave. Also in the accordingly modified treatment device 10, the configuration of the treatment device 10 described above is employed, and the same configuration is denoted by the same reference numeral, and detailed description thereof is not repeated.
   In this case, the treatment device 10 applies an electromagnetic wave to a site to be treated. The treatment device 10 includes file holder 13 that holds file 11 (antenna) to be located in a site to be treated, high-frequency signal generating circuit 19 that supplies file 11 (antenna) with electric power so as to apply an electromagnetic wave from file 11 (antenna), and control circuit 21 that controls a frequency of the electromagnetic wave to be applied from file 11 (antenna). Control circuit 21 controls the frequency of the electromagnetic wave to be applied from file 11 (antenna) to fall within a range of 1.001 MHz to 11.700 MHz. Preferably, the frequency of the electromagnetic wave to be applied is controlled to fall within a range of 7.751 ± 2 MHz. In the treatment device 10, file 11 (antenna) functions as a monopole antenna.
(b) The configuration of the treatment device 10 is not limited to the configuration having file holder 13 for attachment of file 11, and capable of measuring a root canal length and passing a high frequency current as described above, but may be combined with a configuration of motor-driving a treatment tool, a configuration of sonically driving a treatment tool or the like.
(c) While the part of tooth 900 where bone defect 905 or a root apex lesion is generated has been described as a site to be treated where bone regeneration is conducted by means of treatment device 10, a part where a bone defect or a lesion is generated other than tooth 900 may be a site to be treated without limited to the above.

It is to be understood that the above description is illustrative, but not restrictive in every respect. The scope of the present disclosure is only defined by the claimed subject-matter, and includes all modifications within the claimed subject-matter.

## Claims

1. A treatment device (10) that is configured to pass a high frequency current through a bone defect (905) generated in a root canal treatment, (905), the treatment device (10) comprising:
a holder (13) that holds an electrode (11) to be located at the bone defect (905);
a power supply unit (19) that is configured to pass a high frequency current through the electrode (11); and
a controller (21) that is configured to control a frequency of the high frequency current to be passed through the electrode (11) from the power supply unit (19),
the controller (21) controlling the frequency of the high frequency current to be passed through the electrode (11) to fall within a range of 1.001 MHz to 11.700 MHz; wherein
the high frequency current is configured to promote bone regeneration in the bone defect (905).

2. The treatment device (10) according to claim 1, wherein
the controller (21) is configured to control the frequency of the high frequency current to be passed through the electrode (11) to fall within a range of 7.751 ± 2 MHz.

3. The treatment device (10) according to claim 1 or claim 2, wherein
the controller (21) is configured to control a waveform of the high frequency current to be passed through the electrode (11) to be a sinusoidal wave.

4. The treatment device (10) according to any one of the preceding claims, wherein
the controller (21) is configured to control a current value of the high frequency current to be passed through the electrode (11) to be less than 20 mA.

5. The treatment device (10) according to any one of the preceding claims, wherein
the controller (21) is capable of switching between a first control mode of passing a high frequency current having a frequency controlled to fall within a first range of 1.001 MHz to 11.700 MHz through the electrode (11), and a second control mode of passing a high frequency current having a frequency controlled to fall within a second range that is different from the first range through the electrode (11).

6. The treatment device (10) according to claim 5, wherein
the controller (21) is configured to divide one energization period that passes a high frequency current through the electrode (11) into a plurality of periods, and the energization period includes:
a first period of passing a high frequency current having a frequency controlled to fall within the second range through the electrode (11), and
a second period of passing a high frequency current having a frequency controlled to fall within the first range through the electrode (11).

7. The treatment device (10) according to claim 5 or claim 6, wherein
the controller (21) is capable of varying a frequency of the high frequency current within the first range or the second range, and is capable of varying a current value of the high frequency current within a range set in accordance with the first range or the second range.

## Patentansprüche

1. Behandlungsvorrichtung (10), die konfiguriert ist, um einen Hochfrequenzstrom durch einen Knochendefekt (905) zu leiten, der in einer Wurzelkanalbehandlung erzeugt wird, wobei die Behandlungsvorrichtung (10) Folgendes aufweist:
einen Halter (13), der eine Elektrode (11) hält, die an dem Knochendefekt (905) angeordnet werden soll;
eine Stromversorgungseinheit (19), die konfiguriert ist, um einen Hochfrequenzstrom durch die Elektrode (11) zu leiten; und
eine Steuerung (21), die konfiguriert ist, um eine Frequenz des Hochfrequenzstroms zu steuern, der durch die Elektrode (11) von der Stromversorgungseinheit (19) geleitet werden soll,
wobei die Steuerung (21) die Frequenz des Hochfrequenzstroms steuert, der durch die Elektrode (11) geleitet werden soll, um in einen Bereich von 1,001 MHz bis 11,700 MHz zu fallen; wobei
der Hochfrequenzstrom konfiguriert ist, um eine Knochenregeneration in dem Knochendefekt (905) zu fördern.

2. Behandlungsvorrichtung (10) nach Anspruch 1, wobei
die Steuerung (21) konfiguriert ist, um die Frequenz des Hochfrequenzstroms zu steuern, der durch die Elektrode (11) geleitet werden soll, um in einen Bereich von 7,751 ± 2 MHz zu fallen.

3. Behandlungsvorrichtung (10) nach Anspruch 1 oder Anspruch 2, wobei
die Steuerung (21) konfiguriert ist, um eine Wellenform des Hochfrequenzstroms zu steuern, der durch die Elektrode (11) geleitet werden soll, um eine Sinuswelle zu sein.

4. Behandlungsvorrichtung (10) nach einem der vorhergehenden Ansprüche, wobei
die Steuerung (21) konfiguriert ist, um einen Stromwert des Hochfrequenzstroms zu steuern, der durch die Elektrode (11) geleitet werden soll, um weniger als 20 mA zu sein.

5. Behandlungsvorrichtung (10) nach einem der vorhergehenden Ansprüche, wobei
die Steuerung (21) in der Lage ist, zwischen einem ersten Steuermodus des Leitens eines Hochfrequenzstroms mit einer Frequenz, die gesteuert wird, um in einen ersten Bereich von 1,001 MHz bis 11,700 MHz zu fallen, durch die Elektrode (11) und einem zweiten Steuermodus des Leitens eines Hochfrequenzstroms mit einer Frequenz, die gesteuert wird, um in einen zweiten Bereich zu fallen, der sich von dem ersten Bereich unterscheidet, durch die Elektrode (11) umzuschalten.

6. Behandlungsvorrichtung (10) nach Anspruch 5, wobei
die Steuerung (21) konfiguriert ist, um eine Erregungsperiode, die einen Hochfrequenzstrom durch die Elektrode (11) leitet, in eine Vielzahl von Perioden zu unterteilen, und die Erregungsperiode umfasst:
eine erste Periode des Leitens eines Hochfrequenzstroms mit einer Frequenz, die gesteuert wird, um in den zweiten Bereich zu fallen, durch die Elektrode (11) und
eine zweite Periode des Leitens eines Hochfrequenzstroms mit einer Frequenz, die gesteuert wird, um in den ersten Bereich zu fallen, durch die Elektrode (11).

7. Behandlungsvorrichtung (10) nach Anspruch 5 oder Anspruch 6, wobei
die Steuerung (21) in der Lage ist, eine Frequenz des Hochfrequenzstroms innerhalb des ersten Bereichs oder des zweiten Bereichs zu variieren, und in der Lage ist, einen Stromwert des Hochfrequenzstroms innerhalb eines Bereichs zu variieren, der gemäß dem ersten Bereich oder dem zweiten Bereich eingestellt ist.

## Revendications

1. Dispositif de traitement (10) qui est configuré pour faire passer un courant haute fréquence
à travers un défaut osseux (905) généré lors d'un traitement de canal radiculaire,
le dispositif de traitement (10) comprenant :
un dispositif de maintien (13) qui maintient une électrode (11) à placer au niveau du défaut osseux (905) ;
une unité d'alimentation électrique (19) qui est configurée pour faire passer un courant haute fréquence à travers l'électrode (11) ; et
un contrôleur (21) qui est configuré pour commander une fréquence du courant haute fréquence à faire passer à travers l'électrode (11) à partir de l'unité d'alimentation électrique (19),
le contrôleur (21) contrôlant la fréquence du courant haute fréquence à faire passer à travers l'électrode (11) pour qu'elle s'inscrive dans une plage de 1001 MHz à 11 700 MHz ; dans lequel
le courant haute fréquence est configuré pour favoriser la régénération osseuse dans le défaut osseux (905).

2. Dispositif de traitement (10) selon la revendication 1, dans lequel
le contrôleur (21) est configuré pour commander la fréquence du courant haute fréquence à faire passer à travers l'électrode (11) pour qu'elle s'inscrive dans une plage de 7751 ± 2 MHz.

3. Dispositif de traitement (10) selon la revendication 1 ou la revendication 2, dans lequel
le contrôleur (21) est configuré pour commander une forme d'onde du courant haute fréquence à faire passer à travers l'électrode (11) pour qu'elle soit une onde sinusoïdale.

4. Dispositif de traitement (10) selon l'une des revendications précédentes, dans lequel
le contrôleur (21) est configuré pour commander une valeur de courant du courant haute fréquence à faire passer à travers l'électrode (11) pour qu'elle soit inférieure à 20 mA.

5. Dispositif de traitement (10) selon l'une des revendications précédentes, dans lequel
le contrôleur (21) est capable de commuter entre un premier mode de commande consistant à faire passer un courant haute fréquence ayant une fréquence commandée pour s'inscrire dans une première plage de 1001 MHz à 11 700 MHz à travers l'électrode (11), et un second mode de commande consistant à faire passer un courant haute fréquence ayant une fréquence commandée pour s'inscrire dans une seconde plage qui est différente de la première plage à travers l'électrode (11).

6. Dispositif de traitement (10) selon la revendication 5, dans lequel
le contrôleur (21) est configuré pour diviser une période d'excitation qui fait passer un courant haute fréquence à travers l'électrode (11) en une pluralité de périodes, et la période d'excitation inclut :
une première période de passage d'un courant haute fréquence ayant une fréquence commandée pour s'inscrire dans la seconde plage à travers l'électrode (11), et
une seconde période de passage d'un courant haute fréquence ayant une fréquence commandée pour s'inscrire dans la première plage à travers l'électrode (11).

7. Dispositif de traitement (10) selon la revendication 5 ou la revendication 6, dans lequel
le contrôleur (21) est capable de faire varier une fréquence du courant haute fréquence dans la première plage ou la seconde plage, et est capable de faire varier une valeur de courant du courant haute fréquence dans une plage fixée en conformité avec la première plage ou la seconde plage.
